# EUROPÄISCHE PATENTANMELDUNG

(11) **EP 1 090 988 A1**
(43) Veröffentlichungstag der Anmeldung: **11.04.2001**
(21) Anmeldenummer: 99119404.4
(22) Anmeldetag: 30.09.1999
(51) Int. Cl.: C12N 9/88, C12N 15/60, C07K 14/26, C12Q 1/00

(54) **Verfahren zur rekombinanten Herstellung von Holo-Citratlyase**

(71) Anmelder: Roche Diagnostics GmbH, 68298 Mannheim (DE); ETH Zürich, 8092 Zürich (CH)
(72) Erfinder: Dimroth, Peter, Prof., 8700 Küsnacht (CH); Bott, Michael, Prof., 52428 Jülich (DE); Schneider, Karin, 8051 Zürich (CH)

(57) **Zusammenfassung**

Verfahren zur Herstellung eines Proteins mit Citratlyase-Aktivität indem ein geeignetes Plasmid in einem Wirtsorganismus exprimiert wird und das Protein in aktiver Form isoliert wird, dadurch gekennzeichnet, daß das Plasmid die Informtion von einem aus mindestens sechs Genen bestehenden Gencluster sowie einen induzierbaren Promotor aufweist. Ferner betrifft die Erfindung die Verwendung des rekombinanten Enzyms und einen entsprechenden Testkit zur Bestimmung von Zitronensäure.

SEQUENCE LISTING

<110> Roche Diagnostics GmbH

<120> Verfahren zur rekombinanten Herstellung von
      Holo-Citratlyase

<130> 523400EP

<140>
<141>

<160> 7

<170> PatentIn Ver. 2.1

<210> 1
<211> 36
<212> DNA
<213> E. coli

<400> 1
ccctctagag aacaacattc gttgcaaatc gataac                                36

<210> 2
<211> 38
<212> DNA
<213> E. coli

<400> 2
ccgcgaattc ttagttccac atggcgagaa tcggccag                              38

<210> 3
<211> 5484
<212> DNA
<213> E. coli

<400> 3
gaacaacatt cgttgcaaat cgataacaac atgcaccttc aggatactat ttattatgtt 60
cggcaatgat attttcaccc gcgtaaaacg ttcagaaaat aaaaaaatgg cggaaatcgc 120
ccaattcctg catgaaaatg atttgagcgt tgacaccaca gtcgaagtat ttattaccgt 180
aacccgcgat gaaaagctta tcgcgtgcgg tggaattgcc ggaaatatta ttaaatgcgt 240
tgctatcagt gaatccgtcc gcggtgaagg actggcgctg acattagcca ctgaattgat 300
aaacctcgcc tatgagcggc acagcacgca tctgtttatt tataccaaaa ccgaatacga 360
ggcgctgttc cgccagtgcg gtttttccac gctgaccagc gtacccggcg tgatggtgct 420
gatggaaaac agcgccacgc gactgaaacg ctatgccgaa tcgctgaaaa aatttcgtca 480
tccagggaac aagattggct gcattgtgat gaacgccaat ccctttacga atggtcaccg 540
ttatctgatt caacaggctg cggcacagtg cgactggttg catctgtttt tagtcaaaga 600
agattcttca cgcttcccct atgaagaccg gctggatttg gtgttaaaag gcaccgccga 660
tattccacgc ctgactgtgc atcgtggctc cgaatacatc atctcccgcg ctacgttccc 720
ttgctacttc attaaagaac agagcgtcat taaccattgt tacaccgaaa ttgatctgaa 780
gattttccgt cagtacctcg ctcccgcgct gggtgtaact caccgctttg tcggtactga 840
acccttttgt cgcgttaccg cccagtacaa ccaggatatg cgctactggc tggaaacgcc 900
gactatctcc gcaccgccca tcgaactggt tgaaattgag cggctgcgtt accaggagat 960
gccgatatcc gcttcccggg tacgtcaact gctggcgaaa aacgatctca cggctatcgc 1020
gccgctggtc cctgcagtca cgctgcatta tttgcagaac ctgcttgagc actcccgcca 1080
ggacgcggca gctcgtcaaa agacccccgc atgagaaaca ggtgaaaaat gaaaataaac 1140
cagcccgccg ttgcaggcac ccttgagtct ggggatgtga tgatacgcat cgccccactc 1200
gatacgcagg atatcgacct gcaaatcaat agcagcgttg agaaacagtt tggcgatgca 1260

attcgcacca ccattctgga cgttctcgcc cgctacaacg tgcgcggcgt acagctgaat 1320
gtcgatgaca aaggcgcact ggactgcatt ttacgtgcac gactggaagc cctgctggca 1380
cgcgccagcg gtatcccggc tctgccatgg gaggattgcc aatgatttcc gcttcgctgc 1440
aacaacgtaa aactcgcacc cgccgcagca tgttgtttgt gcctggtgcc aatgccgcga 1500
tggtcagcaa ctccttcatc tacccggctg atgccctgat gtttgacctc gaagactccg 1560
tagcattgcg tgaaaaagac accgcccgcc gcatggttta ccacgcgctg caacatccgc 1620
tgtatcgcga tattgaaacc attgtgcgtg tcaacgcgct ggattccgaa tggggtgtta 1680
acgacctgga agccgtcgtt cgcggtggtg cggacgttgt gcgtctgccg aaaaccgata 1740
ccgctcagga tgttctggat attgaaaaag agatcctgcg tatcgaaaaa gcctgtggtc 1800
gtgaacccgg cagcaccggc ctgctggcgg cgattgaatc tccgctgggg attacccgcg 1860
cagtggaaat cgctcacgct tccgagcgtt tgatcggtat cgccctcggt gcagaagact 1920
atgtgcgcaa cctgcgtaca gaacgctccc cggaaggaac tgaactgctg ttcgcacgct 1980
gttccatttt gcaggccgcg cgctctgcgg gtattcaggc gttcgatacc gtctattccg 2040
acgctaacaa cgaagccgga tttctgcaag aagccgccca catcaaacag ctgggctttg 2100
acggcaaatc gctgatcaac ccgcgtcaga ttgatctgct gcacaacctc tacgcaccga 2160
cccagaaaga agtggatcac gcccgccgcg tcgtagaagc cgctgaagcc gccgctcgcg 2220
aaggcctcgg cgtggtttcc ctgaacggca agatggtgga cggtccggtt atcgatcgcg 2280
cccgtctggt gctctcccgt gcagaacttt ccggcatccg cgaagaataa ggcaatcaaa 2340
atgacgcaga aaattgaaca atctcaacga caagaacggg tagcggcctg gaatcgtcgc 2400
gctgaatgcg atcttgccgc tttccagaac tcgccaaagc aaacctacca ggctgaaaaa 2460
gcgcgcgatc gcaaactgtg cgccaacctg gaagaagcga ttcgtcgctc tggtttacag 2520
gacggcatga cggtttcctt ccatcacgct ttccgtggcg gtgacctgac cgtcaatatg 2580
gtgatggacg tcatcgcgaa gatgggcttt aaaaacctga ccctggcgtc cagctccctg 2640
agtgattgcc atgcgccgct ggtagaacac attcgccagg gcgtggttac ccgcatttat 2700
acctccggcc tgcgtggtcc actggcggaa gagatctccc gtggtctgct ggcagaaccg 2760
gtgcagatcc actctcacgg cggtcgtgtg catctggtac agagcggcga actgaatatc 2820
gacgtggctt tcctcggcgt cccgtcctgt gatgaattcg gtaatgccaa cggctacacc 2880
ggtaaagcct gctgcggctc cctcggctat gcaatagttg atgccgacaa cgcaaaacag 2940
gtcgtgatgc ttaccgaaga actgctgcct tatccgcata atccggcaag cattgagcaa 3000
gatcaggttg atttgatcgt caaagttgac cgcgttggcg atgctgcaaa aatcggcgct 3060
ggcgcgaccc gtatgaccac taacccgcgc gaactgctta ttgcccgtag cgctgcggat 3120
gtgattgtca actctggcta cttcaaagaa ggtttctcca tgcaaaccgg caccggcggc 3180
gcatcgctgg cggtaacccg tttcctggaa gacaaaatgc gtagccgcga tattcgcgcc 3240
gacttcgccc ttggcggtat taccgcgacg atggttgacc tgcacgaaaa aggtctgatc 3300
cgcaaactgc tggatgtgca gagctttgac agccatgctg cgcaatcgct ggcccgtaac 3360
cccaatcaca tcgaaatcag cgccaaccag tacgctaact ggggttcgaa aggcgcatcg 3420
gttgatcgtc tcgacgtggt ggtactgagc gcgctggaaa ttgacaccca gttcaacgtt 3480
aacgtgctga ccggctctga cggcgtactg cgtggtgctt ccggtggtca ctgcgatacc 3540
gcgattgcct ctgcgctttc catcatcgtc gcgccgctgg tacgcggtcg tattccgact 3600
ctggtggata acgtactgac ctgcatcacc ccaggctcca gtgtcgatat tctggtcaca 3660
gaccacggta tcgcagttaa cccggcacgt ccggaactgg cagaacgtct gcaggaagcg 3720
ggcattaaag tggtttccat tgagtggctg cgcgaacgtg cgcgtctgct gaccggtgaa 3780
ccacagccga ttgaattcac agaccgcgtc gttgccgttg tgcgttaccg cgatggctcg 3840
gtgatcgatg ttgtgcatca ggtgaaggaa taagccatgc acctgcttcc tgaactcgcc 3900
agccaccatg cggtatcaat tcccgagctg ctcgtcagcc gggatgaaag gcaagcacgg 3960
caacacgtct ggctcaagcg ccatcctgtt ccactggtct cctttaccgt ggttgcgcct 4020
gggccgatta aagacagcga ggtcacacgc cgaattttta atcatggcgt gacagccttg 4080
cgtgccttag ccgcaaaaca gggctggcaa attcaggagc aggctgcact ggtttccgcc 4140
agcgggccgg agggcatgtt gagcattgcc gccccggctc gcgacctcaa gctcgccacc 4200
attgagcttg aacatagtca tcctctcggg cggttatggg atatcgatgt cctgacgccc 4260
gaaggcgaaa ttctctcccg ccgcgactat tcactgccgc ctcgccgctg cctgttgtgc 4320
gaacaaagcg cagccgtctg cgcgcgtgga aaaacccatc aactgaccga tttactcaac 4380
cgcatggagg cactgctgaa cgatgtcgat gcctgcaacg tcaactaaaa ccacaaagct 4440
tgcgacgtca ttaatcgatg agtacgccct gctgggctgg cgcgccatgc tgactgaagt 4500
caatctgtca ccgaaaccag gcctcgtgga tcgcattaac tgcggtgcgc acaaagatat 4560
ggcgctggaa gatttccacc gcagcgcgct ggcgattcag ggctggctac cccgtttcat 4620
tgaatttggt gcctgtagtg cggaaatggc accagaagcg gtactccacg gattacgccc 4680
aattggtatg gcttgcgaag gtgatatgtt ccgcgccact gcgggcgtaa acacgcataa 4740
aggcagcatt ttttctttag ggctgctatg tgcggcaatt ggccgtttgc ttcaactcaa 4800

ccaaccggta acgccaacaa ccgtttgttc tacggcggca agtttctgcc gtggcctgac 4860
cgatcgcgaa ctgcgtacca ataattcaca actgacggca ggtcaacggt tgtaccaaca 4920
gcttggcctt accggcgcac gcggtgaagc cgaagcgggt tatccactgg tgatcaatca 4980
cgccttgccg cattacctca ctctgctgga tcaggggtta gatcctgaac tggcattgct 5040
cgataccttg ctcctactga tggcgatcaa cggcgatacc aacgttgcat cgcgcggtgg 5100
cgaggggggc ctgcgctggc tacagcgcga ggcgcaaaca ttattgcaaa aagggggcat 5160
tcgaaccccc gccgatctcg attatctccg gcagttcgac agggagtgta tcgaacgaaa 5220
tctcagtcca ggcggcagtg ctgacctact gatccttacc tggtttttag cacagattta 5280
attatttaag cacttgataa atttggaaat attaattttc ggagaacccg tatgtcttta 5340
gcaaaagata atatatggaa actattggcc ccactggtgg tgatgggtgt catgtttctt 5400
atccctgtcc ccgacggtat gccgccgcag gcatggcatt acttcgctgt gtttgtggca 5460
atgattgtcg gcatgatcct cgag 5484

<210> 4
<211> 33
<212> DNA
<213> E. coli

<400> 4
aaatttcata tgcacctgct tcctgaactc gcc 33

<210> 5
<211> 36
<212> DNA
<213> E. coli

<400> 5
gggcccctcg agttagttga cgttgcaggc atcgac 36

<210> 6
<211> 552
<212> DNA
<213> E. coli

<400> 6
atgcacctgc ttcctgaact cgccagccac catgcggtat caattcccga gctgctcgtc 60
agccgggatg aaaggcaagc acggcaacac gtctggctca agcgccatcc tgttccactg 120
gtctccttta ccgtggttgc gcctgggccg attaaagaca gcgaggtcac acgccgaatt 180
tttaatcatg gcgtgacagc cttgcgtgcc ttagccgcaa aacagggctg gcaaattcag 240
gagcaggctg cactggtttc cgccagcggg ccggagggca tgttgagcat tgccgccccg 300
gctcgcgacc tcaagctcgc caccattgag cttgaacata gtcatcctct cgggcggtta 360
tgggatatcg atgtcctgac gcccgaaggc gaaattctct cccgccgcga ctattcactg 420
ccgcctcgcc gctgcctgtt gtgcgaacaa agcgcagccg tctgcgcgcg tggaaaaacc 480
catcaactga ccgatttact caaccgcatg gaggcactgc tgaacgatgt cgatgcctgc 540
aacgtcaact aa 552

<210> 7
<211> 5593
<212> DNA
<213> Klebsiella pneumoniae

<400> 7
ttaattaaca acataaaaac cataaagcca attaagccac gagaaaaact gtgacttaaa 60
tacaagaatc catagccgaa cgctggcgaa atacagttcg ttttgaaatg acgaagcgct 120
aaaaaatgac actgatatta aaacgcgttc agctattaaa agataaaccg cggcgagagg 180
cgatcgatcg gtttctccgc cagcatcaac tgtcgttaga ggccgactgc gaaatggcga 240
ttatcgccga gtatcagcag cggctggtcg gctgcggtgc tatcgccggc aatgtgctga 300
aatgcatcgc catcgatccc tcgctgcagg gggaggggct gagccttaaa ttactgaccg 360
agctcctgac gctggcctat gagctggggc gcagcgaact gtttttgttc actaaacctt 420

gcaatgccgc gttattttcc ggcgccggct tctggccgat agcccaggcg ggcgaccgcg 480
ccgtgctaat ggaaaatagc cgcgaacggc tgactcgtta ctgtcgacag ctggcgatgt 540
accgtcagcc gggaagaaaa atcggcgcta tcgtgatgaa tgctaatcca ttcaccctcg 600
gccaccgctg gttggtagaa caggcggcca gccagtgcga ctggctgcat ctgtttgtgg 660
tcaaagaaga tgcgtcctgc ttttcctatc acgatcgctt caagctcatt gaacagggga 720
ttaccggcat cgataaggtg acgctgcatc ccggttcggc gtatctgatc tcgcgggcga 780
cgttccccgg ctatttcctg aaagagcagg gggtggttga tgactgccac agccagattg 840
acctgcagct cttccgcgag cgcctggccc cggcgctgca gattacccat cgctttgtcg 900
gcaccgagcc gctgtgtccc ctgacccgta attacaacca gcgcatgaag tcactactgg 960
aagcgccagg cgacgcgccg cccattgaag tagttgagct tgcgcgaatc gaaaaaaatg 1020
gtggacccgt gtcggcctcc cgagtgcgcg aactctatcg acagcgcaac tggcaggcgg 1080
tcgcggcgct ggtaccgccg ggaaccctct cttttctgat gcaactggcg gaaagcgaac 1140
atcaaaccgc ctgatttata cgccctaact aaggattttc ccctatggaa atgaagattg 1200
acgccctggc cggcacgctg gagtccagcg atgtgatggt caggattgga cccgcggcgc 1260
agccgggcat tcagctggaa atcgacagca ttgtgaaaca acagtttggc gctgcgattg 1320
agcaggtagt gagagaaacg ctggctcagc ttggcgtgaa acaggccaac gtggtggtcg 1380
atgataaagg cgcgctggaa tgtgttttgc gagctcgcgt acaggccgcg gcgctgcgcg 1440
cggcgcaaca gacccaatta caatggagcc agctatgaaa ccacgtcgca gtatgttgtt 1500
catccctggc gccaatgccg ccatgttaag cacgtcattc gtctacggcg ctgatgctgt 1560
gatgttcgac ctggaagatg ccgtttcgct gcgcgagaaa gataccgctc gtctgctggt 1620
gtatcaggcg ctgcagcatc cactgtatca ggatatcgaa accgtggtgc gtattaaccc 1680
gctaaatacc ccgtttggtc tggccgatct ggaagccgtg gttcgtgcgg gcgtggatat 1740
ggtgcgtctg ccgaaaaccg acagcaaaga agatatccat gagctggaag cgcatgttga 1800
gcggattgaa cgcgagtgcg gccgggaagt gggcagcacc aagttaatgg cggcgatcga 1860
gtcggcgctg ggcgtggtga acgcggtgga aatcgcccgc gccagcccgc gtctggcggc 1920
gatcgcgctg gcggccttcg attacgtgat ggatatgggc acctcccgcg gcgacggtac 1980
tgaactgttc tacgcccgct gcgctgtact gcatgccgcc cgcgttgccg gcatcgccgc 2040
ctatgacgtg gtgtggtcgg atatcaataa tgaagagggc ttcctggcgg aagcgaatct 2100
ggccaaaaac ctcggcttta acggcaaatc gttggttaac ccacgacaaa ttgaactcct 2160
gcatcaggtc tatgccccga cgcgcaaaga ggtcgatcac gcgctggaag tgattgccgc 2220
ggcggaagaa gccgaaacgc gaggtctggg tgtggtatcg ctgaacggca agatgatcga 2280
tggaccgatt atcgaccatg ctcgcaaagt ggtggcgctc tcggcttccg gtattcgtga 2340
ttaaggggaa taagatgaaa gagacagtag caatgcttaa tcagcagtac gtgatgccga 2400
atggactgac accttatgcc ggcgtaacgg cgaaaagtcc ctggctggcg agtgagagcg 2460
aaaagcgcca gcgcaaaatc tgcgattcgc tggaaacggc aatccgtcgc tccggcctgc 2520
aaaacggcat gaccatctcg tttcaccacg cgtttcgcgg cggtgacaaa gtcgtcaata 2580
tggtagtggc gaagctggcg gaaatgggtt ttcgcgatct caccctggcg tccagttcgc 2640
tgatcgacgc ccactggccg ctgatcgagc atattaaaaa tggcgtgatc cgccagatct 2700
acacctccgg cctgcgcggc aagttgggcg aggagatctc cgccggttta atggaaaacc 2760
cggtgcagat ccactcccac ggcggtcgcg tacagctgat tcaaagcggc gagctgtcga 2820
ttgatgtcgc gtttctcggc gttccttgct gcgatgagtt tggcaacgcc aacggcttta 2880
gcggtaaatc acgctgcggt tctctgggct acgcgcgcgt cgatgccgag cacgctaaat 2940
gcgtggtgct gctcaccgaa gagtgggtgg attatcctaa ctatccggcc agtattgccc 3000
aggatcaggt ggatctgata gtccaggtag atgaagtcgg cgatccgcaa aaaattaccg 3060
cgggtgccat ccgtctgacc agcaacccgc gcgagctgct gatcgcccgc caggcggcga 3120
aagtcgttga gcactccggt tactttaaag agggtttctc gctgcagacc ggtaccggcg 3180
gcgcctcgct ggcagtaact cgcttccttg aagataaaat gcgccgtaac ggcattaccg 3240
ccagcttcgg cctcggcggt atcaccggga cgatggtcga tttgcacgaa aaagggttga 3300
tcaaaacgct gctcgatacc cagtccttcg atggtgacgc ggcgcgttcg ctggcgcaga 3360
acccgaacca tgtcgagatc tccaccaatc agtatgccag cccgggctcc aaaggcgcct 3420
cctgcgagcg cttaaacgtg gtgatgctca gcgcgctgga aattgatatc gactttaacg 3480
ttaacgtgat gaccggttct aacggtgtgc tgcgcggggc gtccggtggc catagcgata 3540
ccgccgccgg tgcggatttg accattatta ccgcgccgtt agttcgcggc cgtattccct 3600
gcgtcgtgga aaaggtgctg acccgcgtca cgccgggggc cagcgtggat gtgctggtca 3660
ctgaccacgg cattgcggtc aacccggcac gtcaggacct gatcgacaat ttgcgcagcg 3720
caggcattcc gctgatgacc attgaggaac tgcagcagcg tgctgagctg ttgactggca 3780
agccgcagcc gatcgaattc accgatcggg tggtggcggt ggtgcgctat cgcgacggtt 3840
cggtcatcga tgtgattcgt caggtgaaaa acagcgacta aacgcagagg ggaaaggcca 3900
tgagcgacgt gttaattaat cctgcgcgtg tgcggcgcgt gaagccactg agtgccgaag 3960

aggtggtcag cgcggtagag cgcgcgctgt tgaccgaagt tcgcctgacc ccaaagcccg 4020
ggttggtgga tattcgtaac gctggcgcgc actgggatat ggatctggcc tcgtttgagg 4080
ccagcaccgc ggtggtggct ccgtggatgg agaaattttt catcatgggc cacgatactg 4140
cggcggtcgc gccggagcag gtattgatga tgctgcgccc ggtagggatg gcctgtgaga 4200
acgatatgct ggaggccacc ggcggggtga atacccatcg cggggcgatc ttcgcttttg 4260
gcctgctcag cgcggcggcg ggcaggctgg tgtcgaaagg tgagccgata gagcagcacc 4320
ggctttgcga ccaggtggcg cgcttctgtc gcggcatggt tatgcaggag ttgtcttctg 4380
ctggcgggga acggctcagt aaaggcgagg ctcattttct acgctatggt ctctccgggg 4440
cccgcggcga ggcggagagc ggtttcctga cggtgcgtac ccaggccatg ccagtcttta 4500
cccgcatgat ggaagagacc ggcgacagta atctggcgct actgcaaacc ctgctgcatc 4560
tgatggcgtg gaatgatgac accaacctgg tctcgcgcgg cgggcttgcc gggctgaact 4620
ttgtccagca ggaggcgcag cgactgctgt ggcagggcgg cgtgctggcg gacggcgggc 4680
tggaggcgct gcgacagttt gacgatgagc tgattgcccg ccatctcagc cctggcggca 4740
gcgccgatct gttggcggtg acctggtttt tatccgcgtt tcccgccggc gcgcttttcc 4800
cgctgtaacc cactgcaata ccgccttcgc ccgcactgta cgggcgaggg cgccatcatt 4860
agccttcccg gttgtcatcc ggtaaacacg gaatcgcggc acaatcgtat agtttttact 4920
gatatcgtcc gccgtttgtc ataaatttct aattatcggc gtttttgagt agcggcccgc 4980
tgacgggctg gttactctga aaacaattta cgtaatgtta acaaaagaga atagctatgc 5040
atgatgcaca aatccgcgtg gccatcgccg gcgcgggcgg ccggatggga cgccagttaa 5100
ttcaggctgc attgcagatg gaaggcgtgg cgctgggcgc ggcgctggag cgcgaagggt 5160
caagcctggt gggcagcgac gccggcgagc tggcgggcgc cggcaaagcg ggcgtcgcgg 5220
tgcagagcag cctggcggcg gtaaaagatg atttcgacgt gttgatcgat tttacccgcc 5280
cggaaggcac gctgaaccat ctggcgtttt gccgcgagca cggcaaaggg atggtcatcg 5340
gcaccaccgg ttttgacgac gctggcaaac aggcgattcg cgatgccgcg caggacattg 5400
ccattgtctt cgccgctaac tttagcgttg gcgtcaatgt cctgttgaag ctgctggaga 5460
aggcggcgaa ggtgatgggc gactataccg acatcgaaat tatcgaagcg caccaccggc 5520
ataaagtgga tgcgccgtca ggcaccgcgc tggcgatggg cgaagcgatc gccggggcat 5580
tgaacaaaga tct 5593

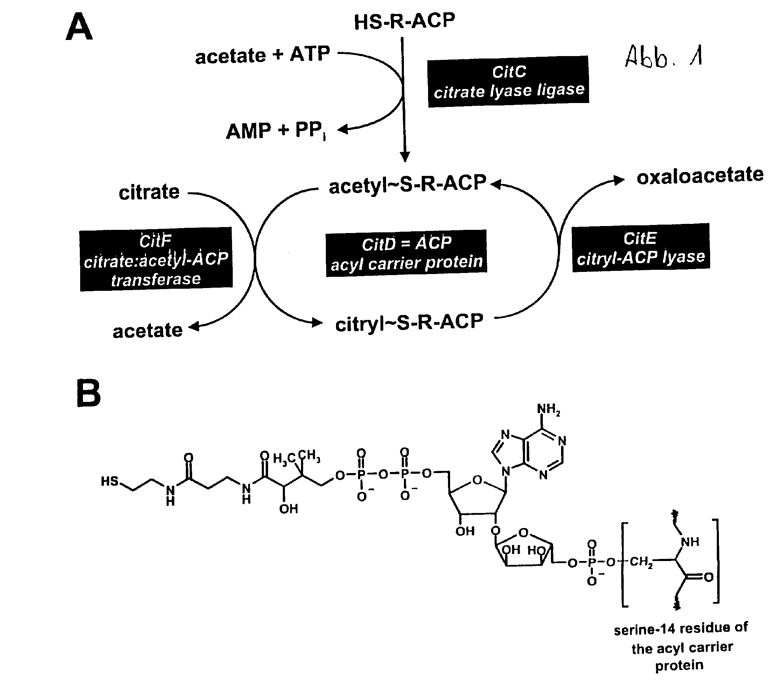

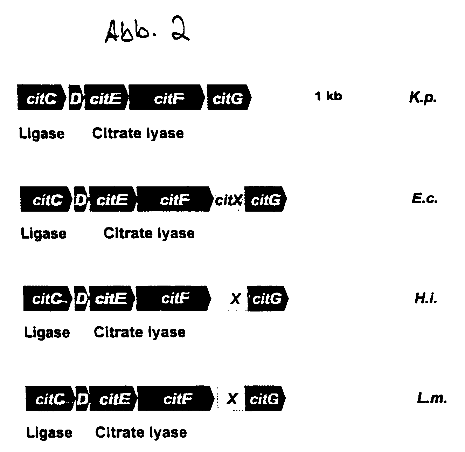

## Beschreibung

Das Enzym Citratlyase (EC 4.1.3.6) gilt als Schlüsselenzym des anaeroben Citrat-Abbaus und kann dementsprechend aus einer Anzahl unterschiedlicher prokaryontischer Zellen isoliert werden. Das Enzym katalysiert die Spaltung von Citrat zu Acetat und Oxalacetat. Darüber hinaus ist bekannt, daß der Enzymkomplex des heute am besten untersuchten Citratlyase-Enzyms aus Klebsiella pneumoniae (vormals: Klebsiella aerogenes) sich aus jeweils sechs Kopien drei verschiedener Untereinheiten, und zwar eine α-, β- und γ-Untereinheit, zusammensetzt und ein Molekulargewicht von etwa 550.000 Dalton aufweist. Ferner ist bekannt, daß das katalytisch aktive Zentrum in der α- und β-Untereinheit lokalisiert ist, während die γ-Untereinheit die Bindungsstelle für die prosthetische Gruppe 2'-(5"-phophoribosyl)-3'-dephospho CoA besitzt. Diese prosthetische Gruppe ist über eine Phosphordiesterbindung an den Serinrest 14 gebunden.

Das Citratlyase-Enzym wird für die meisten Anwendungen, die vornehmlich in der klinischen Chemie und der Lebensmittelanalytik liegen, in hoher Reinheit benötigt. Daher wird angestrebt, das Enzym in aktiver Form durch rekombinante Methoden in bestimmten Wirtszellen überzuproduzieren und aus diesen zu isolieren. Ein derartiges Verfahren ist noch nicht beschrieben oder in anderer Weise bekannt gemacht worden. Üblicherweise wird daher Citratlyase heutzutage aus Klebsiella pneumoniae-Zellen isoliert, die unter anaeroben Bedingungen mit Citrat als einziger Kohlenstoff- und Energiequelle gezüchtet wurden. Die Citratlyase-Gene von Klebsiella pneumoniae wurden kloniert und sequenziert (M. Bott und P. Dimroth, Mol. Microbiol. Vol. 14, 347-356 (1994)). Diese Gene sind Teil des citC-Operons, das aus den fünf Genen citCDEFG besteht. Das citC-Gen kodiert für Citratlyase-Ligase, welche die Bildung eines Acetylthioesters katalysiert. Die Gene citD, citE und citF kodieren für die gamma-, beta- und alpha-Untereinheit der Citratlyase. Das durch citG kodierte Protein ist an der Biosynthese der prosthetischen Gruppe beteiligt. Ferner ist bekannt, daß das citC-Operon in Abwesenheit von Sauerstoff und in Gegenwart von Citrat sowie Na⁺-Ionen induziert wird; darüber hinaus ist die Expression streng abhängig vom citA/citB-Regulationssystem (M. Bott et al., Mol. Microbiol. Vol. 18, 533-546 (1995); M. Meyer et al., J. Mol. Biol. Vol. 269, 719-731 (1997)).

Die Expression der für Citratlyase kodierenden Gene aus Klebsiella pneumoniae, welche aus praktischen Überlegungen bevorzugt in prokaryontischen Zellen, wie z.B. E. coli erfolgen würde, resultiert in einer inaktiven, wenn auch löslichen Form des Enzyms (M. Bott und P. Dimroth, Mol. Microbiol. Vol. 14, 347-356 (1994)). Durch anschließende Zugabe von Acetyl-Coenzym A, das als Substituent für den Acetyl-Thioester der nativen prosthetischen Gruppe 2'-(5"-phosphoribosyl)-3'-dephospho CoA bekannt ist, kann das rekombinante Apo-Citratlyase-Enzym zum Holo-Enzym aktiviert werden (M. Bott und P. Dimroth, Mol. Microbiol. : 14(2), 347-356 (1994)). Eine solche zusätzliche Aktivierungsmaßnahme ist jedoch umständlich und aufwendig. Darüber hinaus ist der zwingende Zusatz von Acetyl-CoA für den kommerziellen Vertrieb von Citratlyase bzw. der Apo-Form ungeeignet, da die Substanz bei längerer Lagerung bei 4°C zerfällt.

Aufgabe der zugrunde liegenden Erfindung ist daher, eine rekombinante, lösliche und zugleich aktive Holo-Citratlyase zur Verfügung zu stellen, womit die Nachteile der bekannten Maßnahmen ausgeräumt werden.

Gelöst wird die Aufgabe durch ein Verfahren zur Herstellung eines Proteins mit Citratlyase-Aktivität, indem ein geeignetes Plasmid in einem Wirtsorganismus exprimiert wird und das Plasmid die Information von einem aus mindestens sechs Genen bestehenden Gencluster sowie einen induzierbaren Promotor aufweist. Die den Gencluster ausmachenden Gene kodieren für bestimmte Untereinheiten des Proteins mit Citratlyase-Aktivität und/oder für an der Biosynthese des vollständigen Enzyms beteiligte Komponenten. Insbesondere enthält ein geeignetes Plasmid die Gene citC, citD, citE, citF, citG und ein beispielsweise aus E. coli erhältliches DNA-Fragment, welches zwischen den Genen citF und citG auf dem E. coli Citratlyase-Gencluster lokalisiert ist. Die Gene citD, citE und citF kodieren für die entsprechenden γ-, β- und α-Untereinheiten des Enzyms und weisen Molekulargewichte von etwa 11.000 Dalton, 32.000 Dalton bzw. 55.000 Dalton auf. Erfindungsgemäß bevorzugt ist, daß eines der Gene ein DNA-Fragment darstellt, welches für ein das Motif G(A)-R-L-X-D-L(I)-D-V enthaltendes Protein kodiert. Besonders bevorzugt ist ein entsprechendes DNA-Fragment, welches für ein Protein mit einem Molekulargewicht von etwa 20.000 Dalton kodiert.

Weiterhin hat sich als vorteilhaft erwiesen, wenn eines und gegebenenfalls ein weiteres, mit dem ersten Gen fusioniertes Gen der den Gencluster ausmachenden Gene aus einem anderen Organismus stammt als die anderen Gene. Insbesondere hat sich als vorteilhaft erwiesen, wenn das zwischen citF und citG auf dem E. coli Citratlyase-Gencluster lokalisierte DNA-Fragment citX bzw. zu citX homologe Gene aus E. coli, Klebsiella pneumoniae, Haemophilus influenzae oder Leuconostoc mesenteroides und eines oder mehrere der anderen Gene aus dem Mikroorganismus stammen, der für das isolierte Citratlyase-Aktivität aufweisende Protein spezifisch ist, wie beispielsweise Klebsiella pneumoniae. Bei Haemophilus influenza, Leuconostoc mesenteroides (S. Bekal et al., J. Bacteriol. Vol. 180, 647-654 (1998)) und Leuconostoc paramesenteroides (M. Martin et al., FEMS Microbiol. Lett. Vol. 174, 231-238 (1999)) treten die Gene citX und citG fusioniert auf. Entsprechende Fusionsgene weisen somit die Information von zwei Genen auf. Die resultierenden Proteine weisen ein Molekulargewicht von etwa 52.000 Dalton auf und die Aktivitäten von E. coli CitX und CitG, sind also bifunktionell. In Abwesenheit des citX-Gens oder eines zu citG homologen Gens bzw. eines entsprechenden citX-Fusionsgens konnte nach Expression lediglich die niedermolekulare Apo-Form (MG 12.000 Dalton, SDS-PAGE), nicht dagegen die Holo-Form der Citratlyase (MG 14.500 Dalton, SDS-PAGE) detektiert werden.

Als Wirtsorganismus haben sich erfindungsgemäß sowohl Prokaryonten als auch Eukaryonten als geeignet erwiesen. Die Tatsache, daß eine lösliche aktive Citratlyase nunmehr in Prokaryonten, wie z.B. E. coli, auf einfache Weise und in ausreichenden Ausbeuten ohne zusätzliche Aktivierungsmaßnahmen erfolgen kann, ist als besonders vorteilhaft zu werten.

Somit konnte gezeigt werden, daß durch Klonierung des gesamten E. coli citCDEFXG Genclusters unter Kontrolle eines induzierbaren Promotors, wie z.B. lac, lac UV5, T5, tac oder T7-Promotors, ein aktives Enzym mit Citratlyase-Aktivität selbst unter nicht sauerstofflimitierten Bedingungen exprimiert werden kann. Zellextrakte mit entsprechenden Expressionsplasmiden führen zu Citratlyase-Aktivitäten von etwa 4 bis 5 U/mg Protein im zellfreien Extrakt, während Zellen ohne rekombinante Citratlyase bei aeroben Wachstum keine Citratlyase-Aktivität aufweisen.

Darüber hinaus betrifft die Erfindung die gleichzeitige Expression des citCDEFG Genclusters aus Klebsiella pneumoniae und des aus E. coli erhältlichen citX-Gens, wodurch selbst in Prokaryonten, insbesondere in E. coli eine entsprechende Citratlyase in aktiver Form gewonnen werden kann.

Hierbei konnte unter aeroben Wachstumsbedingungen eine Aktivität von etwa 8 U/mg Gesamtprotein im zellfreien Extrakt erreicht werden.

Die Reinigung des Holo-Enzyms erfolgt nach dem Fachmann bekannten Methoden. Aus etwa einem Gramm Zellen (Naßgewicht) können mit dem erfindungsgemäßen Verfahren etwa 100 bis 120 µg lösliches Protein mit Citratlyase-Aktivität gewonnen werden. Die Protein-Bestimmung erfolgte nach P.K. Smith et al., Anal. Biochem. Vol. 150, 76-85 (1985), wobei Ovalbumin als Standard diente. Die spezifische Aktivität der Citratlyase beträgt ca. 70 U/mg Protein (M. Single und P.A. Srere, J. Biol. Chem. Vol. 251(10), 2911-2615 (1976)). Die Aktivität des nach dem erfindungsgemäßen Verfahren erhältlichen Holo-Enzyms ist damit um das ca. 0,5- bis 3-fache höher als die mit Acetyl CoA und Apo-Citratlyase erreichte Aktivität.

Das erfindungsgemäße Verfahren ermöglicht somit, daß erstmals ein rekombinantes lösliches und zugleich aktives Protein mit verbesserter Citratlyase-Aktivität zur Verfügung gestellt wird.

Ferner betrifft die Erfindung einen Test-Kit zur Bestimmung von Zitronensäure, welcher im wesentlichen aus folgenden Komponenten besteht: ein nach dem erfindungsgemäßen Verfahren erhältliches Protein mit Citratlyase-Aktivität, mindestens ein Protein mit wasserstoffübertragender Aktivität, Nicotinamid-adenin-dinukleotid oder ein entsprechendes Derivat in reduzierter Form und gegebenenfalls geeignete Stabilisatoren, Aktivatoren und/oder Substanzen zur Vermeidung bzw. Reduzierung von Störungen, d.h. die eigentliche Reaktion überlagernde oder störende Komponenten bzw. Reaktionen sowie geeignete Pufferlösungen. Als Proteine mit wasserstoffübertragender Aktivität kommen insbesondere L-Malat-Dehydrogenase und L-Lactat-Dehydrogenase in Betracht. Als Stabilisatoren sind prinzipiell solche Substanzen, Zusätze bzw. Maßnahmen geeignet, die den Abbau einer für die Bestimmung wichtigen Eigenschaft bzw. Aktivität helfen zu vermeiden oder zumindest zu verzögern. Der Zusatz von Aktivatoren kann insbesondere bei Vorlage geringer Mengen an Probenmaterial oder stark verdünnter Proben von Vorteil sein.

Ein weiterer Gegenstand der Erfindung ist die Verwendung des rekombinanten löslichen Proteins mit Citratlyase-Aktivität zur Bestimmung von Zitronensäure in der klinischen Chemie, der Lebensmittelanalytik sowie zum Reinheitsnachweis von Kosmetika. In der klinischen Chemie wird ein entsprechender enzymatischer Test vor allem zur Fertilitätsuntersuchung herangezogen und bei der Beobachtung des Therapieverlaufs bei Patienten mit Nierensteinen eingesetzt. In der Lebensmittelanalytik ist die wichtigste Anwendung die Wein- bzw. Fruchtsaftanalytik.

Die enzymatische Methode beruht auf der Spaltung des Citrats durch das Enzym Citratlyase, und zwar in Gegenwart von Mg²⁺-Ionen, zu Oxalacetat und Acetat. In Gegenwart von wasserstoffübertragenden Enzymen, wie z.B. L-Malat-Dehydrogenase und L-Lactat-Dehydrogenase, werden Oxalacetat und dessen Decarboxylierungsprodukt Pyruvat durch reduziertes NADH oder NADPH zu L-Malat und L-Lactat reduziert. Die NADH- bzw. NADPH-Menge ist proportional zur Menge an Citrat und wird bei 334 nm, 340 nm oder 365 nm gemessen.

Die Erfindung betrifft daher auch einen entsprechenden Test-Kit für die Bestimmung von Zitronensäure, der - neben geeigneten Pufferlösungen - ein rekombinantes Protein mit Citratlyase-Aktivität, ein oder mehrere wasserstoffübertragende Enzyme sowie ein Nicotinamid-adenin-dinukleotid oder ein entsprechendes Derivat in reduzierter Form sowie gegebenenfalls geeignete Stabilisatoren, wie beispielsweise Thiolreagenzien aufweist.

### Erläuterungen zu den Abbildungen

### Abbildung 1:

A: Funktion der verschiedenen Untereinheiten in einer durch Citratlyase katalysierten Reaktion und Aktivierung des Enzyms durch Citratlyase-Ligase. HS-R steht für die prosthetische Gruppe.
B: Struktur der prosthetischen Gruppe der Citratlyase 2'-(5"-phosphoribosyl)-3'-phospho-CoA.

### Abbildung 2:

Citratlyase-Gencluster aus Klebsiella pneumoniae (K. p.), Escherichia coli (E. c.), Haemophilus influenzae (H. i.) and Leuconostoc mesenteroides (L. m.) Gensequenzen, die homolog zu E. coli citX sind, sind in leicht grauer Tönung unterlegt.

INFORMATION FOR SEQ ID NO. 1:

SEQUENCE CHARACTERISTICS:
(A) LENGTH : 36 base pairs
(B) TYPE : nucleic acid
(C) STRANDNESS : single
(D) TOPOLOGY : linear

5´- CCCTCTAGAGAACAACATTCGTTGCAAATCGATAAC - 3´

INFORMATION FOR SEQ ID NO. 2:

SEQUENCE CHARACTERISTICS:
(A) LENGTH : 38 base pairs
(B) TYPE : nucleic acid
(C) STRANDNESS : single
(D) TOPOLOGY : linear

5´- CCGCGAATTCTTAGTTCCACATGGCGAGAATCGGCCAG -3´

INFORMATION FOR SEQ ID NO. 3:

SEQUENCE CHARACTERISTICS:
(A) LENGTH : 5484 base pairs
(B) TYPE : nucleic acid
(C) STRANDNESS : single
(D) TOPOLOGY : linear

1 GAACAACATT CGTTGCAAAT CGATAACAAC ATGCACCTTC AGGATACTAT
┌Start citC
51 TTATTATGTT CGGCAATGAT ATTTTCACCC GCGTAAAACG TTCAGAAAAT
101 AAAAAAATGG CGGAAATCGC CCAATTCCTG CATGAAAATG ATTTGAGCGT
151 TGACACCACA GTCGAAGTAT TTATTACCGT AACCCGCGAT GAAAAGCTTA
201 TCGCGTGCGG TGGAATTGCC GGAAATATTA TTAAATGCGT TGCTATCAGT
251 GAATCCGTCC GCGGTGAAGG ACTGGCGCTG ACATTAGCCA CTGAATTGAT
301 AAACCTCGCC TATGAGCGGC ACAGCACGCA TCTGTTTATT TATACCAAAA
351 CCGAATACGA GGCGCTGTTC CGCCAGTGCG GTTTTTCCAC GCTGACCAGC
401 GTACCCGGCG TGATGGTGCT GATGGAAAAC AGCGCCACGC GACTGAAACG
451 CTATGCCGAA TCGCTGAAAA AATTTCGTCA TCCAGGGAAC AAGATTGGCT
501 GCATTGTGAT GAACGCCAAT CCCTTTACGA ATGGTCACCG TTATCTGATT
551 CAACAGGCTG CGGCACAGTG CGACTGGTTG CATCTGTTTT TAGTCAAAGA
601 AGATTCTTCA CGCTTCCCCT ATGAAGACCG GCTGGATTTG GTGTTAAAAG
651 GCACCGCCGA TATTCCACGC CTGACTGTGC ATCGTGGCTC CGAATACATC
701 ATCTCCCGCG CTACGTTCCC TTGCTACTTC ATTAAAGAAC AGAGCGTCAT
751 TAACCATTGT TACACCGAAA TTGATCTGAA GATTTTCCGT CAGTACCTCG
801 CTCCCGCGCT GGGTGTAACT CACCGCTTTG TCGGTACTGA ACCCTTTTGT
851 CGCGTTACCG CCCAGTACAA CCAGGATATG CGCTACTGGC TGGAAACGCC
901 GACTATCTCC GCACCGCCCA TCGAACTGGT TGAAATTGAG CGGCTGCGTT
951 ACCAGGAGAT GCCGATATCC GCTTCCCGGG TACGTCAACT GCTGGCGAAA
1001 AACGATCTCA CGGCTATCGC GCCGCTGGTC CCTGCAGTCA CGCTGCATTA
1051 TTTGCAGAAC CTGCTTGAGC ACTCCCGCCA GGACGCGGCA GCTCGTCAAA
Stop citC┐ ┌Start citD
1101 AGACCCCCGC ATGAGAAACA GGTGAAAAAT GAAAATAAAC CAGCCCGCCG
1151 TTGCAGGCAC CCTTGAGTCT GGGGATGTGA TGATACGCAT CGCCCCACTC
1201 GATACGCAGG ATATCGACCT GCAAATCAAT AGCAGCGTTG AGAAACAGTT
1251 TGGCGATGCA ATTCGCACCA CCATTCTGGA CGTTCTCGCC CGCTACAACG
1301 TGCGCGGCGT ACAGCTGAAT GTCGATGACA AAGGCGCACT GGACTGCATT
1351 TTACGTGCAC GACTGGAAGC CCTGCTGGCA CGCGCCAGCG GTATCCCGGC
Stop citD┐
1401 TCTGCCATGG GAGGATTGCC AATGATTTCC GCTTCGCTGC AACAACGTAA
└Start citE
1451 AACTCGCACC CGCCGCAGCA TGTTGTTTGT GCCTGGTGCC AATGCCGCGA
1501 TGGTCAGCAA CTCCTTCATC TACCCGGCTG ATGCCCTGAT GTTTGACCTC
1551 GAAGACTCCG TAGCATTGCG TGAAAAAGAC ACCGCCCGCC GCATGGTTTA
1601 CCACGCGCTG CAACATCCGC TGTATCGCGA TATTGAAACC ATTGTGCGTG
1651 TCAACGCGCT GGATTCCGAA TGGGGTGTTA ACGACCTGGA AGCCGTCGTT
1701 CGCGGTGGTG CGGACGTTGT GCGTCTGCCG AAAACCGATA CCGCTCAGGA
1751 TGTTCTGGAT ATTGAAAAAG AGATCCTGCG TATCGAAAAA GCCTGTGGTC
1801 GTGAACCCGG CAGCACCGGC CTGCTGGCGG CGATTGAATC TCCGCTGGGG
1851 ATTACCCGCG CAGTGGAAAT CGCTCACGCT TCCGAGCGTT TGATCGGTAT

1901 CGCCCTCGGT GCAGAAGACT ATGTGCGCAA CCTGCGTACA GAACGCTCCC
1951 CGGAAGGAAC TGAACTGCTG TTCGCACGCT GTTCCATTTT GCAGGCCGCG
2001 CGCTCTGCGG GTATTCAGGC GTTCGATACC GTCTATTCCG ACGCTAACAA
2051 CGAAGCCGGA TTTCTGCAAG AAGCCGCCCA CATCAAACAG CTGGGCTTTG
2101 ACGGCAAATC GCTGATCAAC CCGCGTCAGA TTGATCTGCT GCACAACCTC
2151 TACGCACCGA CCCAGAAAGA AGTGGATCAC GCCCGCCGCG TCGTAGAAGC
2201 CGCTGAAGCC GCCGCTCGCG AAGGCCTCGG CGTGGTTTCC CTGAACGGCA
2251 AGATGGTGGA CGGTCCGGTT ATCGATCGCG CCCGTCTGGT GCTCTCCCGT
Stop citE┐ ┌Start citF
2301 GCAGAACTTT CCGGCATCCG CGAAGAATAA GGCAATCAAA ATGACGCAGA
2351 AAATTGAACA ATCTCAACGA CAAGAACGGG TAGCGGCCTG GAATCGTCGC
2401 GCTGAATGCG ATCTTGCCGC TTTCCAGAAC TCGCCAAAGC AAACCTACCA
2451 GGCTGAAAAA GCGCGCGATC GCAAACTGTG CGCCAACCTG GAAGAAGCGA
2501 TTCGTCGCTC TGGTTTACAG GACGGCATGA CGGTTTCCTT CCATCACGCT
2551 TTCCGTGGCG GTGACCTGAC CGTCAATATG GTGATGGACG TCATCGCGAA
2601 GATGGGCTTT AAAAACCTGA CCCTGGCGTC CAGCTCCCTG AGTGATTGCC
2651 ATGCGCCGCT GGTAGAACAC ATTCGCCAGG GCGTGGTTAC CCGCATTTAT
2701 ACCTCCGGCC TGCGTGGTCC ACTGGCGGAA GAGATCTCCC GTGGTCTGCT
2751 GGCAGAACCG GTGCAGATCC ACTCTCACGG CGGTCGTGTG CATCTGGTAC
2801 AGAGCGGCGA ACTGAATATC GACGTGGCTT TCCTCGGCGT CCCGTCCTGT
2851 GATGAATTCG GTAATGCCAA CGGCTACACC GGTAAAGCCT GCTGCGGCTC
2901 CCTCGGCTAT GCAATAGTTG ATGCCGACAA CGCAAAACAG GTCGTGATGC
2951 TTACCGAAGA ACTGCTGCCT TATCCGCATA ATCCGGCAAG CATTGAGCAA
3001 GATCAGGTTG ATTTGATCGT CAAAGTTGAC CGCGTTGGCG ATGCTGCAAA
3051 AATCGGCGCT GGCGCGACCC GTATGACCAC TAACCCGCGC GAACTGCTTA
3101 TTGCCCGTAG CGCTGCGGAT GTGATTGTCA ACTCTGGCTA CTTCAAAGAA
3151 GGTTTCTCCA TGCAAACCGG CACCGGCGGC GCATCGCTGG CGGTAACCCG
3201 TTTCCTGGAA GACAAAATGC GTAGCCGCGA TATTCGCGCC GACTTCGCCC
3251 TTGGCGGTAT TACCGCGACG ATGGTTGACC TGCACGAAAA AGGTCTGATC
3301 CGCAAACTGC TGGATGTGCA GAGCTTTGAC AGCCATGCTG CGCAATCGCT
3351 GGCCCGTAAC CCCAATCACA TCGAAATCAG CGCCAACCAG TACGCTAACT
3401 GGGGTTCGAA AGGCGCATCG GTTGATCGTC TCGACGTGGT GGTACTGAGC
3451 GCGCTGGAAA TTGACACCCA GTTCAACGTT AACGTGCTGA CCGGCTCTGA
3501 CGGCGTACTG CGTGGTGCTT CCGGTGGTCA CTGCGATACC GCGATTGCCT
3551 CTGCGCTTTC CATCATCGTC GCGCCGCTGG TACGCGGTCG TATTCCGACT
3601 CTGGTGGATA ACGTACTGAC CTGCATCACC CCAGGCTCCA GTGTCGATAT
3651 TCTGGTCACA GACCACGGTA TCGCAGTTAA CCCGGCACGT CCGGAACTGG
3701 CAGAACGTCT GCAGGAAGCG GGCATTAAAG TGGTTTCCAT TGAGTGGCTG
3751 CGCGAACGTG CGCGTCTGCT GACCGGTGAA CCACAGCCGA TTGAATTCAC
3801 AGACCGCGTC GTTGCCGTTG TGCGTTACCG CGATGGCTCG GTGATCGATG
Stop citF┐ ┌Start citX
3851 TTGTGCATCA GGTGAAGGAA TAAGCCATGC ACCTGCTTCC TGAACTCGCC
3901 AGCCACCATG CGGTATCAAT TCCCGAGCTG CTCGTCAGCC GGGATGAAAG
3951 GCAAGCACGG CAACACGTCT GGCTCAAGCG CCATCCTGTT CCACTGGTCT
4001 CCTTTACCGT GGTTGCGCCT GGGCCGATTA AAGACAGCGA GGTCACACGC
4051 CGAATTTTTA ATCATGGCGT GACAGCCTTG CGTGCCTTAG CCGCAAAACA
4101 GGGCTGGCAA ATTCAGGAGC AGGCTGCACT GGTTTCCGCC AGCGGGCCGG
4151 AGGGCATGTT GAGCATTGCC GCCCCGGCTC GCGACCTCAA GCTCGCCACC
4201 ATTGAGCTTG AACATAGTCA TCCTCTCGGG CGGTTATGGG ATATCGATGT
4251 CCTGACGCCC GAAGGCGAAA TTCTCTCCCG CCGCGACTAT TCACTGCCGC
4301 CTCGCCGCTG CCTGTTGTGC GAACAAAGCG CAGCCGTCTG CGCGCGTGGA
4351 AAAACCCATC AACTGACCGA TTTACTCAAC CGCATGGAGG CACTGCTGAA
Stop citX┐

4401 CGATGTCGAT GCCTGCAACG TCAACTAAAA CCACAAAGCT TGCGACGTCA
Start citG
4451 TTAATCGATG AGTACGCCCT GCTGGGCTGG CGCGCCATGC TGACTGAAGT
4501 CAATCTGTCA CCGAAACCAG GCCTCGTGGA TCGCATTAAC TGCGGTGCGC
4551 ACAAAGATAT GGCGCTGGAA GATTTCCACC GCAGCGCGCT GGCGATTCAG
4601 GGCTGGCTAC CCCGTTTCAT TGAATTTGGT GCCTGTAGTG CGGAAATGGC
4651 ACCAGAAGCG GTACTCCACG GATTACGCCC AATTGGTATG GCTTGCGAAG
4701 GTGATATGTT CCGCGCCACT GCGGGCGTAA ACACGCATAA AGGCAGCATT
4751 TTTTCTTTAG GGCTGCTATG TGCGGCAATT GGCCGTTTGC TTCAACTCAA
4801 CCAACCGGTA ACGCCAACAA CCGTTTGTTC TACGGCGGCA AGTTTCTGCC
4851 GTGGCCTGAC CGATCGCGAA CTGCGTACCA ATAATTCACA ACTGACGGCA
4901 GGTCAACGGT TGTACCAACA GCTTGGCCTT ACCGGCGCAC GCGGTGAAGC
4951 CGAAGCGGGT TATCCACTGG TGATCAATCA CGCCTTGCCG CATTACCTCA
5001 CTCTGCTGGA TCAGGGGTTA GATCCTGAAC TGGCATTGCT CGATACCTTG
5051 CTCCTACTGA TGGCGATCAA CGGCGATACC AACGTTGCAT CGCGCGGTGG
5101 CGAGGGGGGC CTGCGCTGGC TACAGCGCGA GGCGCAAACA TTATTGCAAA
5151 AAGGGGGCAT TCGAACCCCC GCCGATCTCG ATTATCTCCG GCAGTTCGAC
5201 AGGGAGTGTA TCGAACGAAA TCTCAGTCCA GGCGGCAGTG CTGACCTACT
Stop citG
5251 GATCCTTACC TGGTTTTTAG CACAGATTTA ATTATTTAAG CACTTGATAA
Start citT
5301 ATTTGGAAAT ATTAATTTTC GGAGAACCCG TATGTCTTTA GCAAAAGATA
5351 ATATATGGAA ACTATTGGCC CCACTGGTGG TGATGGGTGT CATGTTTCTT
5401 ATCCCTGTCC CCGACGGTAT GCCGCCGCAG GCATGGCATT ACTTCGCTGT
5451 GTTTGTGGCA ATGATTGTCG GCATGATCCT CGAG

INFORMATION FOR SEQ ID NO. 4:

SEQUENCE CHARACTERISTICS:
(A) LENGTH : 33 base pairs
(B) TYPE : nucleic acid
(C) STRANDNESS : single
(D) TOPOLOGY : linear

5´- AAATTTCATATGCACCTGCTTCCTGAACTCGCC - 3´

INFORMATION FOR SEQ ID NO. 5:

SEQUENCE CHARACTERISTICS:
(A) LENGTH : 36 base pairs
(B) TYPE : nucleic acid
(C) STRANDNESS : single
(D) TOPOLOGY : linear

5´- GGGCCCCTCGAGTTAGTTGACGTTGCAGGCATCGAC - 3´

INFORMATION FOR SEQ ID NO. 6:

SEQUENCE CHARACTERISTICS:
(A) LENGTH : 553 base pairs
(B) TYPE : nucleic acid
(C) STRANDNESS : single
(D) TOPOLOGY : linear

1 ATGCACCTGC TTCCTGAACT CGCCAGCCAC CATGCGGTAT CAATTCCCGA
51 GCTGCTCGTC AGCCGGGATG AAAGGCAAGC ACGGCAACAC GTCTGGCTCA
101 AGCGCCATCC TGTTCCACTG GTCTCCTTTA CCGTGGTTGC GCCTGGGCCG
151 ATTAAAGACA GCGAGGTCAC ACGCCGAATT TTTAATCATG GCGTGACAGC
201 CTTGCGTGCC TTAGCCGCAA AACAGGGCTG GCAAATTCAG GAGCAGGCTG
251 CACTGGTTTC CGCCAGCGGG CCGGAGGGCA TGTTGAGCAT TGCCGCCCCG
301 GCTCGCGACC TCAAGCTCGC CACCATTGAG CTTGAACATA GTCATCCTCT
351 CGGGCGGTTA TGGGATATCG ATGTCCTGAC GCCCGAAGGC GAAATTCTCT
401 CCCGCCGCGA CTATTCACTG CCGCCTCGCC GCTGCCTGTT GTGCGAACAA
451 AGCGCAGCCG TCTGCGCGCG TGGAAAAACC CATCAACTGA CCGATTTACT
501 CAACCGCATG GAGGCACTGC TGAACGATGT CGATGCCTGC AACGTCAACT
551 AA

INFORMATION FOR SEQ ID NO. 7:

SEQUENCE CHARACTERISTICS:
(A) LENGTH : 5593 base pairs
(B) TYPE : nucleic acid
(C) STRANDNESS : single
(D) TOPOLOGY : linear

1 TTAATTAACA ACATAAAAAC CATAAAGCCA ATTAAGCCAC GAGAAAAACT GTGACTTAAA
61 TACAAGAATC CATAGCCGAA CGCTGGCGAA ATACAGTTCG TTTTGAAATG ACGAAGCGCT
Start citC
121 AAAAAATGAC ACTGATATTA AAACGCGTTC AGCTATTAAA AGATAAACCG CGGCGAGAGG
181 CGATCGATCG GTTTCTCCGC CAGCATCAAC TGTCGTTAGA GGCCGACTGC GAAATGGCGA
241 TTATCGCCGA GTATCAGCAG CGGCTGGTCG GCTGCGGTGC TATCGCCGGC AATGTGCTGA
301 AATGCATCGC CATCGATCCC TCGCTGCAGG GGGAGGGGCT GAGCCTTAAA TTACTGACCG
361 AGCTCCTGAC GCTGGCCTAT GAGCTGGGGC GCAGCGAACT GTTTTTGTTC ACTAAACCTT
421 GCAATGCCGC GTTATTTTCC GGCGCCGGCT TCTGGCCGAT AGCCCAGGCG GGCGACCGCG
481 CCGTGCTAAT GGAAAATAGC CGCGAACGGC TGACTCGTTA CTGTCGACAG CTGGCGATGT
541 ACCGTCAGCC GGGAAGAAAA ATCGGCGCTA TCGTGATGAA TGCTAATCCA TTCACCCTCG
601 GCCACCGCTG GTTGGTAGAA CAGGCGGCCA GCCAGTGCGA CTGGCTGCAT CTGTTTGTGG
661 TCAAAGAAGA TGCGTCCTGC TTTTCCTATC ACGATCGCTT CAAGCTCATT GAACAGGGGA
721 TTACCGGCAT CGATAAGGTG ACGCTGCATC CCGGTTCGGC GTATCTGATC TCGCGGGCGA
781 CGTTCCCCGG CTATTTCCTG AAAGAGCAGG GGGTGGTTGA TGACTGCCAC AGCCAGATTG
841 ACCTGCAGCT CTTCCGCGAG CGCCTGGCCC CGGCGCTGCA GATTACCCAT CGCTTTGTCG
901 GCACCGAGCC GCTGTGTCCC CTGACCCGTA ATTACAACCA GCGCATGAAG TCACTACTGG
961 AAGCGCCAGG CGACGCGCCG CCCATTGAAG TAGTTGAGCT TGCGCGAATC GAAAAAAATG
1021 GTGGACCCGT GTCGGCCTCC CGAGTGCGCG AACTCTATCG ACAGCGCAAC TGGCAGGCGG
1081 TCGCGGCGCT GGTACCGCCG GGAACCCTCT CTTTTCTGAT GCAACTGGCG GAAAGCGAAC
Stop citC                                                                                    Start citD
1141 ATCAAACCGC CTGATTTATA CGCCCTAACT AAGGATTTTC CCCTATGGAA ATGAAGATTG

1201 ACGCCCTGGC CGGCACGCTG GAGTCCAGCG ATGTGATGGT CAGGATTGGA CCCGCGGCGC
1261 AGCCGGGCAT TCAGCTGGAA ATCGACAGCA TTGTGAAACA ACAGTTTGGC GCTGCGATTG
1321 AGCAGGTAGT GAGAGAAACG CTGGCTCAGC TTGGCGTGAA ACAGGCCAAC GTGGTGGTCG
1381 ATGATAAAGG CGCGCTGGAA TGTGTTTTGC GAGCTCGCGT ACAGGCCGCG GCGCTGCGCG
Stop citD
1441 CGGCGCAACA GACCCAATTA CAATGGAGCC AGCTATGAAA CCACGTCGCA GTATGTTGTT
Start citE
1501 CATCCCTGGC GCCAATGCCG CCATGTTAAG CACGTCATTC GTCTACGGCG CTGATGCTGT
1561 GATGTTCGAC CTGGAAGATG CCGTTTCGCT GCGCGAGAAA GATACCGCTC GTCTGCTGGT
1621 GTATCAGGCG CTGCAGCATC CACTGTATCA GGATATCGAA ACCGTGGTGC GTATTAACCC
1681 GCTAAATACC CCGTTTGGTC TGGCCGATCT GGAAGCCGTG GTTCGTGCGG GCGTGGATAT
1741 GGTGCGTCTG CCGAAAACCG ACAGCAAAGA AGATATCCAT GAGCTGGAAG CGCATGTTGA
1801 GCGGATTGAA CGCGAGTGCG GCCGGGAAGT GGGCAGCACC AAGTTAATGG CGGCGATCGA
1861 GTCGGCGCTG GGCGTGGTGA ACGCGGTGGA AATCGCCCGC GCCAGCCCGC GTCTGGCGGC
1921 GATCGCGCTG GCGGCCTTCG ATTACGTGAT GGATATGGGC ACCTCCCGCG GCGACGGTAC
1981 TGAACTGTTC TACGCCCGCT GCGCTGTACT GCATGCCGCC CGCGTTGCCG GCATCGCCGC
2041 CTATGACGTG GTGTGGTCGG ATATCAATAA TGAAGAGGGC TTCCTGGCGG AAGCGAATCT
2101 GGCCAAAAAC CTCGGCTTTA ACGGCAAATC GTTGGTTAAC CCACGACAAA TTGAACTCCT
2161 GCATCAGGTC TATGCCCCGA CGCGCAAAGA GGTCGATCAC GCGCTGGAAG TGATTGCCGC
2221 GGCGGAAGAA GCCGAAACGC GAGGTCTGGG TGTGGTATCG CTGAACGGCA AGATGATCGA
2281 TGGACCGATT ATCGACCATG CTCGCAAAGT GGTGGCGCTC TCGGCTTCCG GTATTCGTGA
Stop citE Start citF
2341 TTAAGGGGAA TAAGATGAAA GAGACAGTAG CAATGCTTAA TCAGCAGTAC GTGATGCCGA
2401 ATGGACTGAC ACCTTATGCC GGCGTAACGG CGAAAAGTCC CTGGCTGGCG AGTGAGAGCG
2461 AAAAGCGCCA GCGCAAAATC TGCGATTCGC TGGAAACGGC AATCCGTCGC TCCGGCCTGC
2521 AAAACGGCAT GACCATCTCG TTTCACCACG CGTTTCGCGG CGGTGACAAA GTCGTCAATA
2581 TGGTAGTGGC GAAGCTGGCG GAAATGGGTT TTCGCGATCT CACCCTGGCG TCCAGTTCGC
2641 TGATCGACGC CCACTGGCCG CTGATCGAGC ATATTAAAAA TGGCGTGATC CGCCAGATCT
2701 ACACCTCCGG CCTGCGCGGC AAGTTGGGCG AGGAGATCTC CGCCGGTTTA ATGGAAAACC
2761 CGGTGCAGAT CCACTCCCAC GGCGGTCGCG TACAGCTGAT TCAAAGCGGC GAGCTGTCGA
2821 TTGATGTCGC GTTTCTCGGC GTTCCTTGCT GCGATGAGTT TGGCAACGCC AACGGCTTTA
2881 GCGGTAAATC ACGCTGCGGT TCTCTGGGCT ACGCGCGCGT CGATGCCGAG CACGCTAAAT
2941 GCGTGGTGCT GCTCACCGAA GAGTGGGTGG ATTATCCTAA CTATCCGGCC AGTATTGCCC
3001 AGGATCAGGT GGATCTGATA GTCCAGGTAG ATGAAGTCGG CGATCCGCAA AAAATTACCG
3061 CGGGTGCCAT CCGTCTGACC AGCAACCCGC GCGAGCTGCT GATCGCCCGC CAGGCGGCGA
3121 AAGTCGTTGA GCACTCCGGT TACTTTAAAG AGGGTTTCTC GCTGCAGACC GGTACCGGCG
3181 GCGCCTCGCT GGCAGTAACT CGCTTCCTTG AAGATAAAAT GCGCCGTAAC GGCATTACCG
3241 CCAGCTTCGG CCTCGGCGGT ATCACCGGGA CGATGGTCGA TTTGCACGAA AAGGGTTGA
3301 TCAAAACGCT GCTCGATACC CAGTCCTTCG ATGGTGACGC GGCGCGTTCG CTGGCGCAGA
3361 ACCCGAACCA TGTCGAGATC TCCACCAATC AGTATGCCAG CCCGGGCTCC AAAGGCGCCT
3421 CCTGCGAGCG CTTAAACGTG GTGATGCTCA GCGCGCTGGA AATTGATATC GACTTTAACG
3481 TTAACGTGAT GACCGGTTCT AACGGTGTGC TGCGCGGGGC GTCCGGTGGC CATAGCGATA
3541 CCGCCGCCGG TGCGGATTTG ACCATTATTA CCGCGCCGTT AGTTCGCGGC CGTATTCCCT
3601 GCGTCGTGGA AAAGGTGCTG ACCCGCGTCA CGCCGGGGGC CAGCGTGGAT GTGCTGGTCA
3661 CTGACCACGG CATTGCGGTC AACCCGGCAC GTCAGGACCT GATCGACAAT TTGCGCAGCG
3721 CAGGCATTCC GCTGATGACC ATTGAGGAAC TGCAGCAGCG TGCTGAGCTG TTGACTGGCA
3781 AGCCGCAGCC GATCGAATTC ACCGATCGGG TGGTGGCGGT GGTGCGCTAT CGCGACGGTT
Stop citF
Start
3841 CGGTCATCGA TGTGATTCGT CAGGTGAAAA ACAGCGACTA AACGCAGAGG GGAAAGGCCA
citG
3901 TGAGCGACGT GTTAATTAAT CCTGCGCGTG TGCGGCGCGT GAAGCCACTG AGTGCCGAAG
3961 AGGTGGTCAG CGCGGTAGAG CGCGCGCTGT TGACCGAAGT TCGCCTGACC CCAAAGCCCG

4021 GGTTGGTGGA TATTCGTAAC GCTGGCGCGC ACTGGGATAT GGATCTGGCC TCGTTTGAGG
4081 CCAGCACCGC GGTGGTGGCT CCGTGGATGG AGAAATTTTT CATCATGGGC CACGATACTG
4141 CGGCGGTCGC GCCGGAGCAG GTATTGATGA TGCTGCGCCC GGTAGGGATG GCCTGTGAGA
4201 ACGATATGCT GGAGGCCACC GGCGGGGTGA ATACCCATCG CGGGGCGATC TTCGCTTTTG
4261 GCCTGCTCAG CGCGGCGGCG GGCAGGCTGG TGTCGAAAGG TGAGCCGATA GAGCAGCACC
4321 GGCTTTGCGA CCAGGTGGCG CGCTTCTGTC GCGGCATGGT TATGCAGGAG TTGTCTTCTG
4381 CTGGCGGGGA ACGGCTCAGT AAAGGCGAGG CTCATTTTCT ACGCTATGGT CTCTCCGGGG
4441 CCCGCGGCGA GGCGGAGAGC GGTTTCCTGA CGGTGCGTAC CCAGGCCATG CCAGTCTTTA
4501 CCCGCATGAT GGAAGAGACC GGCGACAGTA ATCTGGCGCT ACTGCAAACC CTGCTGCATC
4561 TGATGGCGTG GAATGATGAC ACCAACCTGG TCTCGCGCGG CGGGCTTGCC GGGCTGAACT
4621 TTGTCCAGCA GGAGGCGCAG CGACTGCTGT GGCAGGGCGG CGTGCTGGCG GACGGCGGGC
4681 TGGAGGCGCT GCGACAGTTT GACGATGAGC TGATTGCCCG CCATCTCAGC CCTGGCGGCA
4741 GCGCCGATCT GTTGGCGGTG ACCTGGTTTT TATCCGCGTT TCCCGCCGGC GCGCTTTTCC
Stop citG
4801 CGCTGTAACC CACTGCAATA CCGCCTTCGC CCGCACTGTA CGGGCGAGGG CGCCATCATT
4861 AGCCTTCCCG GTTGTCATCC GGTAAACACG GAATCGCGGC ACAATCGTAT AGTTTTTACT
4921 GATATCGTCC GCCGTTTGTC ATAAATTTCT AATTATCGGC GTTTTTGAGT AGCGGCCCGC
4981 TGACGGGCTG GTTACTCTGA AAACAATTTA CGTAATGTTA ACAAAAGAGA ATAGCTATGC
5041 ATGATGCACA AATCCGCGTG GCCATCGCCG GCGCGGGCGG CCGGATGGGA CGCCAGTTAA
5101 TTCAGGCTGC ATTGCAGATG GAAGGCGTGG CGCTGGGCGC GGCGCTGGAG CGCGAAGGGT
5161 CAAGCCTGGT GGGCAGCGAC GCCGGCGAGC TGGCGGGCGC CGGCAAAGCG GGCGTCGCGG
5221 TGCAGAGCAG CCTGGCGGCG GTAAAAGATG ATTTCGACGT GTTGATCGAT TTTACCCGCC
5281 CGGAAGGCAC GCTGAACCAT CTGGCGTTTT GCCGCGAGCA CGGCAAAGGG ATGGTCATCG
5341 GCACCACCGG TTTTGACGAC GCTGGCAAAC AGGCGATTCG CGATGCCGCG CAGGACATTG
5401 CCATTGTCTT CGCCGCTAAC TTTAGCGTTG GCGTCAATGT CCTGTTGAAG CTGCTGGAGA
5461 AGGCGGCGAA GGTGATGGGC GACTATACCG ACATCGAAAT TATCGAAGCG CACCACCGGC
5521 ATAAAGTGGA TGCGCCGTCA GGCACCGCGC TGGCGATGGG CGAAGCGATC GCCGGGGCAT
5581 TGAACAAAGA TCT

Die folgenden Beispiele erläutern die Erfindung weiter:

### Beispiel 1:

### Kultivierung der Zellen

Es wurden folgende Stämme und Plasmide verwendet: E. coli DH5α bzw. BL21 (DE3) (F.W. Studiar und B.A. Mofatt, J. Mol. Biol. Vol. 189, 113-130 (1986)) und pACYC184 (A.C.Y. Chang et al., J. Bacteriol. Vol. 134, 1141-1156 (1978)). Die E. coli-Zellen wurden routinemäßig in Luria-Bertani (LB)-Medium bei 37°C nach J. Sambrook et al., Molecular Cloning. A Laboratory Manual, Cold Spring Harbor Laboratory Press, Cold Spring Harbor, New York (2nd Edition 1989) kultiviert. Es wurden Antibiotika mit folgenden Endkonzentrationen dazugegeben: Amphicillin 200 µg/ml, Chloramphenicol 50 µg/ml und Kanamycin 50 µg/ml. Der Stamm E. coli DH5a wurde als Wirtsorganismus für die Klonierung verwendet. Die E. coli BL21 (DE3) -Zellen, welche das Phagen T7-Polymerasegen unter Kontrolle eines lacUV5-Promotors enthalten (F.W. Studier und B.A. Moffatt, supra) diente als Wirt für die Expression der Zielgene von pT7-7- und pET-Derivaten. Die Kulturen für die Expression wurden wie folgt hergestellt: Nach Zentrifugation (3000 g, 8 Min.) einer Vorkultur von 40 ml, die über Nacht bei 37°C inkubiert worden war, wurden die Zellen in 20 ml frischem LB-Medium resuspendiert. Die Zellsuspension wurde anschließend zum Animpfen von 2 L desselben Mediums, welches entsprechende Antibiotika enthält, verwendet und die Kultur wurde bei 37°C im Schüttler (180 rpm) inkubiert. Bei Erreichen eines OD₆₀₀-Wertes zwischen 0,5 und 0,8 wurde die Expression der Zielgene durch Zugabe von IPTG (Isopropyl-β-D-thiogalactosid) mit einer Endkonzentration von 1 mM induziert und die Kultur für weitere 3 Stunden bei 37°C im Schüttler (180 rpm) inkubiert. Anschließend wurden die Zellen durch Zentrifugation (30 Min. bei 3000 g) geerntet, einmal mit 20 ml 50 mM Kaliumphosphat, pH 7,0, 1 mM MgCl₂ gewaschen und bei -20°C gelagert.

### Beispiel 2:

### Isolierung der Gene undx Gencluster

Für die Konstruktion des Expressionsplasmides, das den E. coli citCDEFXG-Gencluster enthält, wurde ein 6,9 kb großes Fragment aus der chromosomalen DNA von E. coli via PCR mit den Primern eccl-for (s. SEQ ID NO. 1) und ec-citT-rev (SEQ ID NO. 2) unter Verwendung des Expand High Fidelity PCR Systems von Roche Diagnostics amplifiziert. Das 6.9 kb PCR-Fragment, das zusätzlich das citT-Gen enthält (K.M. Pos et al., J. Bacteriol. Vol. 180, 4160-4165 (1998)), wurde mit den Restriktionsendonukleasen Xbal und Xhol geschnitten und das resultierende 5.5 kb Fragment (SEQ ID NO. 3) und ein ebenfalls entsprechend linearisierten Expressionsvektor, wie z.B. pKK177-3Hb, pKKT5, pUC18, pT7, pET24b auf einem Agarosegel aufgetrennt und die entsprechenden Banden isoliert (QIAEX-Kit der Firma Diagen). Anschließend wurden das PCR-Fragment und das Vektorfragment mittels T4-DNA-Ligase miteinander ligiert. Dazu wurden 1 µl (20ng) Vektorfragment und 3 µl (100 ng) PCR-Fragment, 1 µl 10x Ligase-Puffer (Maniatis et al., 1989 B.27), 1 µl T4-DNA-Ligase, 4 µl steriles H₂O bidest. pipettiert, vorsichtig gemischt und über Nacht bei 16°C inkubiert. Das aus der PCR erhaltene Insert startet 55 bp vor dem citC-Startcodon und endet 203 bp downstream vom citG-Stopcodon.

Für die Konstruktion des Expressionsplasmides, das das citX-Gen aus E. coli enthält (SEQ ID NO. 3), wurde das citX-Gen via PCR aus der chromosomalen DNA mit den Primern ec-citX-for (SEQ ID NO. 4) und ec-citX-rev (SEQ ID NO. 5) unter Verwendung der Pfu DNA Polymerase (Stratagene) amplifiziert. Das Startcodon ist Teil einer NdeI-Restriktionsendonukleaseschnittstelle und direkt hinter dem Stopcodon befindet sich eine XhoI- Restriktionsendonukleaseschnittstelle. Nach Verdau des PCR-Produktes mit NdeI und XhoI wurde das resultierende 555 bp DNA-Fragment (SEQ ID NO. 6) in entsprechend linearisierte Expressionsvektoren ligiert (wie oben beschrieben).

Die Konstruktion des Expressionsplasmides, das den citCDEFG-Gencluster von Klebsiella pneumoniae enthält, ist in M. Bott und P. Dimroth, Molecular Microbiology Vol. 14 (2), 347-356 (1994) beschrieben. Die Sequenz des citCDEFG-Genclusters ist in SEQ ID NO. 7 wiedergegeben.

### Beispiel 3:

### Transformation der unterschiedlichen Expressionsplasmide in verschiedene E. coli Expressionsstämme

Kompetente Zellen verschiedener E. coli-Stämme wurden entsprechend der Methode nach Hanahan (J. Mol. Biol. Vol. 166, 557 ff. (1983)) hergestellt. 200 µl derart hergestellter Zellen wurden mit 20 ng isolierten der entsprechenden Expressionsplasmide versetzt. Nach 30 minütiger Inkubation auf Eis erfolgte ein Hitzeschock (90 Sek. bei 42°C). Anschließend wurden die Zellen in 1 ml LB-Medium überführt und zur phänotypischen Expression 1 Stunde bei 37°C inkubiert. Aliquote dieses Transformationsansatzes wurden auf LB-Platten mit dem entsprechenden Antibiotikum als Selektionsmarker ausplattiert und 15 Stunden bei 37°C inkubiert.

### Beispiel 4:

### Expression der verschiedenen Zielgene

Nach Zentrifugation (3000 g, 8 Min.) von 40 ml Vorkultur, die über Nacht bei 37°C gewachsen worden war, wurden das Zellpellet in 20 ml frischen LB-Medium resuspendiert. Die Zellsuspension wurde daraufhin verwendet, um 2 1 LB-Medium mit den entsprechenden Antibiotika anzuimpfen. Diese Zellkultur wurde bei 37°C im Schüttler (180 rpm) inkubiert. Bei einer optischen Dichte (gemessen bei 600 nm) von 0,5 - 0,8 wurde die Expression der Zielgene durch Zugabe von 1 mM Isopropyl-β-D-thiogalactosid (IPTG, Endkonzentration) induziert und die Kulturen 3 Stunden bei 37°C und 180 rpm weiter inkubiert. Danach wurden die Zellen durch Zentrifugation geerntet (30 Min. bei 3000 g) einmal in 20 ml 50 mM Kaliumphosphat, pH 7,0, gewaschen und bei -20°C eingefroren.

Zur Zellextraktpreparation wurden 1 g Zellen (Naßgewicht) in 4 ml kaltem 50 mM Kaliumphosphat, 1 mM MgCl₂ pH 7,0 resuspendiert. Nach Zugabe des Proteaseinhibitor-Cocktails (Roche Diagnostics) und DNAseI bis zu einer Endkonzentration von 25 mg/ml wurden die Zellen durch eine dreimalige Passage in der French Press bei 108 Mpa aufgeschlossen. Intakte Zellen und Zelltrümmer wurden durch Zentrifugation (30 Min. bei 27000 g) entfernt. Der zellfreie Überstand wurde durch Ultrazentrifugation (1h bei 150000 g) von der Membranfraktion getrennt und der daraus resultierende Zellextrakt kann direkt für enzymatische Studien und zur Proteinreinigung verwendet werden.

### Beispiel 5:

### Citratlyase-Aktivitätstest

Die Citratlyase-Aktivität wurde bei 25°C in einem spektrophotometrischen Test gekoppelt mit Malatdehydrogenase von Roche Diagnostics durchgeführt. Die Testmischung enthielt in einem Endvolumen von 1 ml 50 mM Glycylglycin pH 7,9, 5 mM Kaliumcitrat, 2 mM ZnCl₂, 0,5 mM NADH, 30 U Malatdehydrogenase (Roche Diagnostics) und 10 µl bzw. 20 µl Zellextrakt. Die Oxidation von NADH wurde im Spektrophotometer bei 365 nm gemessen (s = 3,4 mM⁻¹cm⁻¹). Eine Enzymeinheit (Unit) ist definiert als 1 µmol Citrat, welches pro Minute zu Acetat und Oxalacatat abgebaut wird.

## Patentansprüche

1. Verfahren zur Herstellung eines Proteins mit Citratlyase-Aktivität indem ein geeignetes Plasmid in einem Wirtsorganismus exprimiert wird und das Protein in aktiver Form isoliert wird, dadurch gekennzeichnet, daß das Plasmid die Information von einem aus mindestens sechs Genen bestehenden Gencluster sowie einen induzierbaren Promotor aufweist.

2. Verfahren nach Anspruch 1, dadurch gekennzeichnet, daß die Gene für bestimmte Untereinheiten des Proteins mit Citratlyase-Aktivität und/oder für an der Biosynthese des vollständigen Enzyms beteiligte Komponenten kodiert.

3. Verfahren nach einem der Ansprüche 1 oder 2 dadurch gekennzeichnet, daß das Plasmid die Gene citC, citD, citE, citF, citG und ein aus E. coli erhältliches DNA-Fragment, welches zwischen citF und citG auf dem E. coli Citratlyase-Gencluster lokalisiert ist, enthält.

4. Verfahren nach Anspruch 3, dadurch gekennzeichnet, daß das DNA-Fragment für ein 20 kDA großes Protein kodiert.

5. Verfahren nach Anspruch 3 oder 4, dadurch gekennzeichnet, daß das DNA-Fragment für ein das Motif G(A)-R-L-X-D-L(I)-D-V enthaltendes Protein kodiert.

6. Verfahren nach einem der Ansprüche 1 bis 5, dadurch gekennzeichnet, daß mindestens ein Gen aus E. coli, Haemophilus influenzae, Klebsiella pneumoniae oder Leuconostoc mesenteroides erhältlich ist.

7. Verfahren nach einem der Ansprüche 1 bis 6, dadurch gekennzeichnet, daß mindestens vier Gene aus dem Mikroorganismus stammen, der für das isolierte Protein mit Citratlyase-Aktivität spezifisch ist.

8. Verfahren nach Anspruch 7, dadurch gekennzeichnet, daß es sich um Klebsiella pneumoniae handelt.

9. Verfahren nach einem der Ansprüche 1 bis 8, dadurch gekennzeichnet, daß es sich bei dem Wirtsorganismus um einen eukaryontischen oder prokaryontischen Mikroorganismus handelt.

10. Verfahren nach Anspruch 9, dadurch gekennzeichnet, daß es sich um E. coli handelt.

11. Verfahren nach einem der Ansprüche 1 bis 10, dadurch gekennzeichnet, daß die Expression unter aeroben Bedingungen erfolgt.

12. Rekombinantes lösliches Protein mit Citratlyase-Aktivität und einem Molekulargewicht von etwa 14.000 bis 15.000 Dalton erhältlich nach dem Verfahren gemäß einem der Ansprüche 1 bis 11.

13. Test-Kit zur Bestimmung von Zitronensäure, welcher im wesentlichen folgende Komponenten aufweist
(a) ein Protein mit Citratlyase-Aktivität erhältlich nach einem der Ansprüche 1 bis 11,
(b) mindestens ein Protein mit wasserstoffübertragender Aktivität,
(c) Nicotinamid-adenin-dinukleotid oder ein entsprechendes Derivat in reduzierter Form, und
(d) gegebenenfalls geeignete Stabilisatoren, Aktivatoren und/oder Substanzen zur Vermeidung bzw. Reduzierung von Störungen sowie Pufferlösungen.

14. Test-Kit nach Anspruch 13, dadurch gekennzeichnet, daß als wasserstoffübertragende Enzyme L-Malat-Dehydrogenase und gegebenenfalls L-Lactat-Dehydrogenase eingesetzt werden.

15. Verwendung des nach einem der Ansprüche 1 bis 11 erhältlichen Enzyms zur Bestimmung von Zitronensäure.
